Europäisches Patentamt

**European Patent Office**

Office européen des brevets

(11) Publication number : **0 577 266 A2**

(19)

(12) **EUROPEAN PATENT APPLICATION**

(21) Application number : **93304251.7**

(22) Date of filing : **01.06.93**

(51) Int. Cl.$^5$ : **C12P 19/32**

(30) Priority : **01.06.92 US 891593**

(43) Date of publication of application :
**05.01.94 Bulletin 94/01**

(84) Designated Contracting States :
**AT BE CH DE DK ES FR GB GR IE IT LI LU MC NL PT SE**

(71) Applicant : **ICN BIOMEDICALS INC.**
**3300 Hyland Avenue**
**Costa Mesa, California 92626 (US)**

(72) Inventor : **Lin, Paul**
**8521 Terry Drive**
**Huntington Beach, California 92647 (US)**

(74) Representative : **Haigh, Charles Roy et al**
**J.A. KEMP & CO. 14 South Square Gray's Inn**
**London WC1R 5LX (GB)**

(54) **Method for preparing (gamma-32p) labeled nucleotides by enzymatic conversion of dihydroxyacetone phosphate to 3-phosphoglycerate.**

(57)    Carrier-free [$^{32}$P] inorganic phosphate is incorporated into the $\gamma$-phosphate of ATP and other nucleoside triphosphates by a series of enzymatic reactions in the presence of dihydroxyacetone phosphate, NAD$^+$, and their corresponding nucleoside diphosphates. NAD$^+$ is preferably regenerated by malate dehydrogenase and oxaloacetate. The resulting [$\gamma$-$^{32}$P] nucleotides are useful as reagents for analytical determinations. [$\gamma$-$^{32}$P] ATP is also useful for preparing [$\alpha$-$^{32}$P]-labeled nucleotides having high specific activity.

EP 0 577 266 A2

Field of the Invention

The present invention is in the field of biochemistry.

Background of the Invention

A number of phosphotransferase reactions require the production of $[\gamma\text{-}^{32}P]$ATP having high specific activity. Included among these reactions is protein phosphorylation with protein kinases, 5'-OH end labeling of polynucleotides with polynucleotide kinase and the initiation of RNA chains. $[\gamma\text{-}^{32}P]$ ATP is also required for the preparation of $[\alpha\text{-}^{32}P]$-labeled nucleotides for effectively labeling nucleic acids.

Adenosine triphosphate (ATP) and other nucleoside triphosphates labeled with $^{32}P$ in the $\gamma$- phosphate position are generally prepared by enzymatic processes. To date, three procedures have been developed for the production of $[\gamma\text{-}^{32}P]$ nucleoside triphosphates: 1) the Glynn-Chappell method, Biochem. J., 90: 147-149 (1964); 2) the Schendel-Wells method, J. Biol. Chem., 248: 8319-8321 (1973); and 3) the Johnson-Walseth method, Adv. in Cyclic Nucleotide Res., 10: 135-168 (1979). These three methods share a common feature in that selected enzymes and substrates of the glycolytic pathway are used.

In particular, the Glynn-Chappell method involves the exchange of the $\gamma$-phosphate group from unlabeled ATP with $^{32}P$-labeled inorganic phosphate $(P_i)$ in the presence of phosphoglycerate kinase (PKG) and glyceraldehyde 3-phosphate dehydrogenase (GAPDH). Although it is a rather simple procedure, the $[\gamma\text{-}^{32}P]$ATP produced by the Glynn-Chappell Method yields a specific activity of only up to 100 Ci/mmol. The other two methods involve the substrate level phosphorylation of ADP with $^{32}P_i$, resulting in $[\gamma\text{-}^{32}P]$ATP with higher specific activity. The estimated specific activity of $[\gamma\text{-}^{32}P]$ATP produced by the Schendel-Wells and the Johnson-Walseth methods are approximately 1000 and $\geqq4000$ Ci/mmol, respectively, provided that the reagents and conversion enzymes are pure.

The starting material in the Schendel-Wells method is D-glyceraldehyde 3-phosphate which is available commercially only as the diethylacetal form. In this method, the diethylacetal group must first be hydrolyzed and removed leaving D-glyceraldehyde 3-phosphate unprotected. This unprotected substrate is labile in solution, and its degradation increases the amount of unlabeled $P_i$ in the reaction mixture, resulting in decreased specific activity of $[\gamma\text{-}^{32}P]$ATP.

To improve the specific activity of the resulting $[\gamma\text{-}^{32}P]$ATP, Johnson and Walseth used L-$\alpha$-glycerophosphate as the starting material instead of the D-glyceraldehyde 3-phosphate starting material used in the Schendel-Wells method. L-$\alpha$-glycerophosphate, which has proven to be more stable than the starting substrate in the Schendel-Wells method, is first converted by glycerol 3-phosphate dehydrogenase (GPDH) to dihydroxyacetone phosphate, and then to D-glyceraldehyde-3-phosphate by triosephosphate isomerase (TPI). D-glyceraldehyde-3-phosphate, formed as the intermediate, reacts with $^{32}P_i$ via GAPDH to form 1,3-diphosphoglycerate, which then reacts with ADP to produce $[\gamma\text{-}^{32}P]$ATP in the presence of 3-phosphoglycerate kinase (PKG). This process requires the presence of a sufficient amount of $NAD^+$ for the complete incorporation of $^{32}P_i$ into $[\gamma\text{-}^{32}P]$ATP. Therefore, an $NAD^+$ regenerating reaction, catalyzed by pyruvate and L-lactate dehydrogenase (LDH), has been utilized to provide sufficient $NAD^+$ to maintain the forward reaction. The specific activity of $[\gamma\text{-}^{32}P]$ATP produced by the Johnson-Walseth method is variable, being dependent upon the amount of unlabeled $P_i$ contaminating the reagents and enzymes used, as well as the ratio of $^{32}P_i$/unlabeled $P_i$ in the reaction mixture.

The Johnson-Walseth method is also the subject of United States Patent No. 4,209,589. Although this provides higher yield and specific activity than previously attained, it also produces unwanted $^{32}P$-labeled contaminates in the $[\gamma\text{-}^{32}P]$ATP which are undesirable for research purposes. There is therefore a need for a procedure for effectively labeling the $\gamma$-phosphate group of nucleotides (e.g. ATP and GTP) with $^{32}P$, producing $[^{32}P]$ labeled products with a high specific activity, but without producing unwanted $^{32}P$-labeled contaminates.

Summary of the Invention

The present invention involves an enzymatic process for preparing ATP labeled in the $\gamma$-phosphate group with $^{32}P$ using dihydroxyacetone phosphate (DHAP) as the starting material. At present, DHAP is commercially available as the lithium salt, and does not require hydrolysis prior to being used in the reaction. Thus, a much smaller amount of unlabeled phosphate $(P_i)$ is introduced into the reaction mixture as compared to the three processes described above. Although the commercially-available DHAP may contain approximately 5% $P_i$ the resulting $[^{32}P]$-labeled products exhibit a higher specific activity than when D-glyceraldehyde 3-phosphate is used as the starting material as in the Schendel-Wells method. The specific activity of $[^{32}P]$-labeled products derived from the process of the present invention is comparable to that of the Johnson-Walseth method, yet

without the [$^{32}$P] labeled contaminates.

Other unique features of the present invention include:

1. Using dihydroxyacetone phosphate instead of L-α-glycerophosphate as the starting material. Dihydroxyacetone phosphate is commercially available as the lithium salt, which unlike the starting material in the Schendel-Wells method, does not require hydrolysis prior to use. Hence, it is a great improvement over the older diethylacetal derivative. In addition, dihydroxyacetone phosphate is stable at -20°C for months.

2. Removing glycerol 3-phosphate dehydrogenase from the reaction mixture and significantly reducing the amount of lactate dehydrogenase. The concentrations of triosephosphate isomerase and pyruvate kinase used are also much different from the Johnson-Walseth method. Hence, only four conversion enzymes are used in the present invention instead of the five used in the Johnson-Walseth method, which further contributes to the elimination of unwanted $^{32}$P labeled contaminates.

3. Successfully replacing pyruvate and lactate dehydrogenase with oxaloacetate and malate dehydrogenase to support the regeneration of NAD$^+$ from NADH.

4. Determining the reagent concentrations necessary for large-scale commercial production as opposed to the concentrations described by Johnson and Walseth for small-scale academic production.

In the process of the present invention, the DHAP is converted by triosephosphate isomerase (TPI) to D-glyceraldehyde 3-phosphate. The D-glyceraldehyde 3-phosphate is then converted to 1,3-diphosphoglycerate by glyceraldehyde 3-phosphate dehydrogenase (GAPDH) in the presence of $^{32}$P$_i$ and nicotinamide adenine dinucleotide (NAD$^+$). The resulting 1,3-diphosphoglycerate is reacted with the selected nucleoside diphosphate to form the [γ-$^{32}$P] nucleoside triphosphate by means of 3-phosphoglycerate kinase (PKG). All the enzymes used in this process are commercially available and are ready to be used without further purification. The enzymatic reaction is optimized for maximizing the yield of the [γ-$^{32}$P] nucleotide with highest specific activity and with minimum formation of other $^{32}$P-labeled compounds in the reaction mixture. Specifically, the conversion enzymes TPI, GAPDH and PGK are present in concentrations within the range of from about 2 to 10 U/ml, 3 to 10 U/ml, and 3 to 10 U/ml, respectively. Preferably, the concentrations of these enzymes are kept in the low range in order to reduce potential contamination by unlabeled P$_i$ and/or myokinase (adenylate kinase). It has been shown that about 3% to 5% of the $^{32}$P label in the [γ-$^{32}$P] nucleotide preparation could be associated with the β-phosphate group as the result of the presence of myokinase in TRI and GAPDH. Despite the limited amount of conversion enzymes, the process of the present invention surprisingly yields the [γ-$^{32}$P]-labeled products of high specific activity with minimal contamination by $^{32}$P-labeled in the β-phosphate group.

In an optimized reaction, NAD$^+$, which is required for the conversion of D-glyceraldehyde 3-phosphate to 1,3-diphosphoglycerate, is continuously regenerated from its reduced form (NADH) by malate dehydrogenase (MDH) in the presence of oxaloacetate. As oxaloacetate is converted to malate, NADH is oxidized to NAD$^+$. The optimum concentration of MDH is in the range of from about 2 to 10 U/ml. Additionally, the ionic strength of Mg$^{2+}$ and the buffer, tris-(hydroxymethyl) aminomethane (Tris), is optimized not only for maximizing the production of the [γ-$^{32}$P]-labeled nucleotides but also for minimizing the incorporation of $^{32}$P into undesired compounds that are present in the reaction mixtures of the other three processes.

It is a general object of the present invention to provide a method for the production of phosphate-labeled nucleotides.

It is another object of the invention to provide a method for the production of phosphate-labeled nucleotides having high specific activity.

It is a further object of the invention to provide a method for the production of phosphate-labeled nucleotides having high specific activity and minimizing the production of $^{32}$P-labeled contaminates.

These and other objects will become readily apparent to those skilled in the art from the following description and the appended claims.

## Brief Description of the Drawing

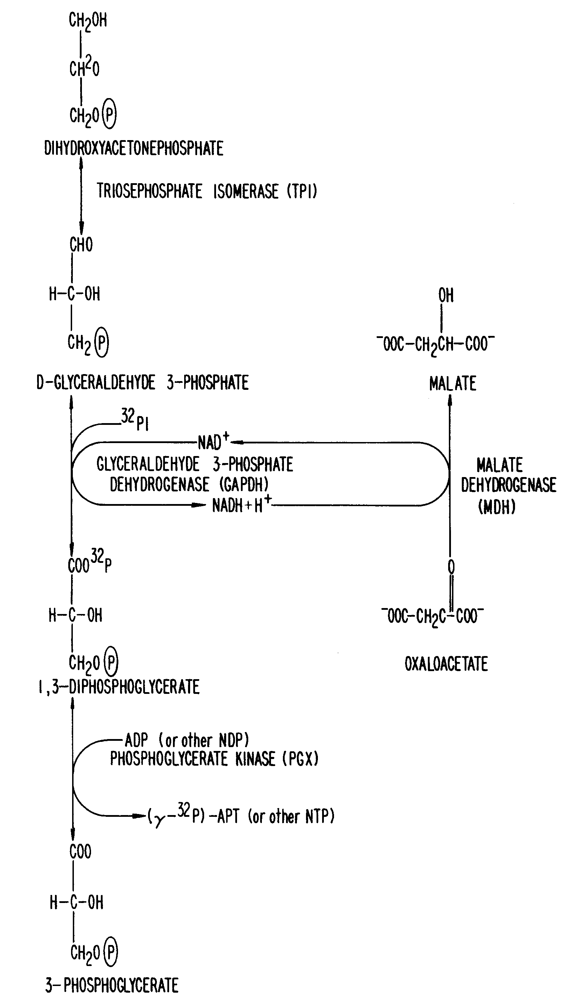

The accompanying drawing (Fig. 1) represents the sequence of enzymatic reactions for incorporating $^{32}$P$_i$ into the γ-phosphate group of a desired nucleotide (e.g. ATP). As illustrated, [γ-$^{32}$P] nucleoside phosphates (NTPs) can be prepared from their corresponding nucleoside diphosphates (NDPs).

## Detailed Description

All the primary reactants are commercially available, namely [$^{32}$P]-inorganic phosphate ($^{32}$P$_i$), dihydroxyacetone phosphate (DHAP) and the nucleoside diphosphates (NDP). These diphosphates and their corresponding triphosphates are summarized below:

| DIPHOSPHATE | TRIPHOSPHATE |
|---|---|
| adenosine 5'-diphosphate (ADP) | adenosine 5'-triphosphate (ATP) |
| 2'-deoxyadenosine 5'-diphosphate (dADP) | 2'-deoxyadenosine 5'-triphosphate (dATP) |
| guanosine 5'-diphosphate (GDP) | guanosine 5'-triphosphate (GTP) |
| cytidine 5'-diphosphate (CDP) | cytidine 5'-triphosphate (CDP) |
| 2'-deoxythymidine 5'-diphosphate (dTDP) | 2'-deoxythimidine 5'-triphosphate (dTTP) |

Since DHAP is commercially available as the lithium salt, it does not need to be hydrolyzed prior to use as does the diethylacetal form of the glyceraldehyde 3-phosphate starting material in the Schendel-Wells method. In addition, if ADP contains a substantial amount of $P_i$, it should be repurified (e.g. chromatography on DEAE-Sephadex) prior to use. The key for producing high specific activity $[\gamma\text{-}^{32}P]$-labeled nucleotides is removing unlabeled $P_i$ from the components in the reaction mixture. Although the concentrations are not critical, the following ranges of initial concentrations are recommended for a high yield of $[\gamma\text{-}^{32}P]$-labeled nucleotides. The concentrations are given in micromoles per liter of reaction mixture.

| Primary Reactant | Concentration ($\mu$M) |
|---|---|
| DHAP | 200-500 |
| NDP | 100-500 |
| $^{32}P_i$ | 0.2-100 |

A suitable amount of DHAP is necessary not only for providing enough D-glyceraldehyde 3-phosphate to react with all of the $^{32}P_i$, but also for limiting the amount of unlabeled $P_i$ (which could be present in the DHAP in as much as 5%) to yield a final product, $[\gamma\text{-}^{32}P]$-labeled nucleotide with high specific activity. Stoichiometrically, a ratio of less than 5 for DHAP/$^{32}P_i$ is desirable, i.e. 5 moles of DHAP per mole of $^{32}P_i$.

The enzymatic process for labeling $^{32}P$ in the $\gamma$-phosphate group of nucleotides requires the presence of $NAD^+$. $NAD^+$ is reduced to NADH by the conversion of D-glyceraldehyde 3-phosphate to 1,3-diphosphoglycerate. Since it is a reversible reaction, a high ratio of $NAD^+$/NADH is needed to keep the reaction going forward, which can be accomplished by oxidation of the NADH formed back to $NAD^+$ in the presence of oxaloacetate and malate dehydrogenase (MDH) as represented in Fig. 1. The oxidation of NADH also can be carried out with pyruvate and lactate dehydrogenase (LDH), as described by Johnson and Walseth. Notably, however, effective $NAD^+$ regeneration is required for complete incorporation of $^{32}P_i$ into the $\gamma$-phosphate group of the desired nucleotide. Yet an excess amount of $NAD^+$ added to the reaction mixture without the regenerate system results in only a partial incorporation of the $^{32}P_i$. To obtain a complete reaction, the following initial concentrations of reactants are recommended:

| Reactant | Concentration |
|---|---|
| $\beta$-$NAD^+$ | 1-5 mM |
| Oxaloacetic acid | 0.2-1 mM |
| MDH | 2-10 U/ml |

The oxaloacetic acid can be added as the free acid and the $NAD^+$ solution is preferably neutralized with sodium hydroxide prior to use.

Only four enzymes are used in the process of this invention and their activities in the reaction mixture, expressed as International Units (U), are summarized below. A unit (International Activity Standard) is defined as that amount of enzyme activity necessary to incorporate 1 micromole of $^{32}P_i$ into $[\gamma\text{-}^{32}P]$-labeled nucleotide per minute under optimal reaction conditions.

| Enzyme | Concentration (U/ml) |
|--------|---------------------|
| TPI | 2-10 |
| GAPDH | 3-10 |
| PGK | 3-10 |
| MDH | 2-10 |

Using minimal amounts of enzymes and a reasonable incubation period to complete the reaction will reduce the contamination by myokinase (MK) found particularly in the commercial preparations of TPI and GAPDH (e.g. Boehringer-Mannheim). As discussed above, myokinase activity in these enzyme preparations can result in as much as 3% to 5% [$\beta$-$^{32}$P] labeling in [$\gamma$-$^{32}$P] nucleotide production. For example, using about 2 U/ml of TPI and about 3 U/ml of GAPDH in combination with a larger amount of PGK (about 8 U/ml) and MDH (about 8 U/ml) has been effective in minimizing the effect of myokinase contamination.

The overall enzymatic reaction should be carried out under conditions which are suitable not only for high yield of [$^{32}$P]-labeled nucleotide production, but also for minimal incorporation of $^{32}$P$_i$ into undesired components (or compounds other than a desired nucleotide) in the reaction mixture. For example, there are at least two unidentified [$^{32}$P]-labeled compounds found in the reaction mixture of [$\gamma$-$^{32}$P] ATP production, which could account for up to 10% of the total $^{32}$P depending upon reaction conditions. These two components could be associated with the enzyme preparations or the reactants used. To minimize the production of these two components and maintain both high yield and high purity of [$\gamma$-$^{32}$P]-labeled nucleotide production, the reaction is preferably conducted under high pH and high ionic strength. Although the reaction can take place at pH ranging from about 7.0 to 10, pH 9 is generally used for production. It is noteworthy that this pH is also suitable for the subsequent reaction of polynucleotide kinase when [$\gamma$-$^{32}$P] ATP is used for preparing nucleotides labeled with $^{32}$P in the $\alpha$-phosphate group. While the PGK reaction requires a divalent cation such as magnesium, it has been found that the overall reaction for [$\gamma$-$^{32}$P] nucleotide production can proceed effectively without any addition of $Mg^{2+}$. Presumably, there is enough divalent cation(s) associated with the commercial enzyme preparations and/or the reactants (e.g. ADP) used in the reaction to supply the necessary cations for the reaction to proceed. Nevertheless, a high concentration of $MgCl_2$ ($\sim$50 mM) should be used for the overall reaction in order to reduce the amount of $^{32}$P$_i$ incorporated into the undesired components indicated above. Additionally, the conversion enzymes are stabilized by the presence of a hydrosulful reagent such as dithiothreitol or cysteine at concentrations of from about 5 to 30 mM.

Under the conditions described above, the reaction may be conducted at room temperature (20-25°C), although temperature conditions may range from about 10 to 37°C. The reaction generally proceeds rapidly and reaches completion ($\geqq$95% of $^{32}$P$_i$ incorporation) in about 15 to 20 minutes. The incubation period can be prolonged for up to 1 hour without significant breakdown of the [$\gamma$-$^{32}$P] nucleotides formed. The progress of the incorporation of $^{32}$P$_i$ into the desired nucleotide can be easily monitored by thin-layer chromatography. A small aliquot of about 0.5 $\mu$l is applied to a PEI-cellulose thin-layer strip which is then developed in 0.3 M $KH_2PO_4$ (pH 7.0) for about 10 to 30 minutes. The strip is then exposed to an x-ray film and subsequently developed. The resulting autoradiogram indicates the relative amount of $^{32}$P incorporation into the desired nucleotide. $^{32}$P$_i$ and [$^{32}$P]-labeled nucleotide can be separated by the above thin-layer chromatographic system. Upon completion, the reaction mixture can be heat treated at about 100°C for about 2 minutes to inactivate the enzymes. Usually, a small amount of [$\gamma$-$^{32}$P] nucleotide is broken down during the heat treatment.

The resulting [$\gamma$-$^{32}$P]-labeled nucleotide can be recovered and purified by known chromatographic procedures. For instance, anion-exchange chromatography (e.g. DEAE-Sephadex or Dowex-1 in either Cl$^-$ HCOO$^-$ form) is very useful for separation of the nucleotides from their corresponding nucleoside diphosphates and nucleoside monophosphates. The sample can be preferentially eluted from the anion-exchange column by a continuous or discontinuous gradient of increasing salt concentration. High-performance liquid chromatography can be used to further purify the product.

The [$\gamma$-$^{32}$P] nucleotides are useful for a number of analytic determinations. For example, [$\gamma$-$^{32}$P] ATP can be used directly without any purification for end-labeling ribonucleic acid or for reactions (e.g. polynucleotide kinase) to produce other [$^{32}$P]-labeled compounds. The highly purified [$\gamma$-$^{32}$P] nucleotides are also needed for the study of a number of phosphotransferase reactions, i.e., protein phosphorylation with protein kinase, 5'-OH end-labeling of nucleic acids with polynucleotide kinase, and initiation of ribonucleic acid chains with ribonucleic acid polymerases. In addition, [$\gamma$-$^{32}$P] ATP can be used for assaying the activity of ATPase. Besides protein kinase, [$\gamma^{32}$P] GTP can be used for labeling the 5'-end of ribonucleic acid as well as for the assay of

hormone-sensitive GTPase. [$\gamma^{32}$P]-labeled dATP, CTP and dTTP also can be used for studying phosphotransfer reactions of a variety of enzymes. This invention is further presented by the following specific examples.

Example I

A. Incorporation of $^{32}$P$_i$ into [$\gamma$-$^{32}$P] ATP. The synthesis of [$\gamma$-$^{32}$P]ATP from $^{32}$P$_i$ is carried out by the reactions shown in Fig. 1. The typical production consists of 0.5 volume of $^{32}$P$_i$ (up to 500 mCi), 0.06 volume of the enzyme mix (see Table I below) and 0.6 volume of the reagent mixture (see Table II below). The final concentrations of enzymes and reagents are also shown in Tables I and II. The reaction is initiated by the addition of the reagent mixture to the $^{32}$P$_i$ solution and immediately followed by the addition of enzyme mix. After incubation at room temperature for approximately 20 min (or until >95% of the $^{32}$P$_i$ has been incorporated into [$\gamma$-$^{32}$P]ATP), the reaction is terminated by setting the reaction vessel in boiling water for about 2 to 3 minutes.

## Table I

| Enzyme[a] | Enzyme Mix | | |
| | Stock mixture[b] ($\mu$l) | Enzyme-cysteine mixture[c] (U/ml) | Concentration in the reaction[d] ($\mu$g/ml   U/ml) |
|---|---|---|---|
| Triosephosphate isomerase (Rabbit muscle; 5000 U/mg; 2 mg/ml) | 1.0 | 36.4 | 0.3   1.9 |
| Glyceraldehyde-3-phosphate dehydrogenase (Rabbit muscle; 80 U/mg; 10 mg/ml) | 20 | 58.2 | 37.5   3.0 |
| 3-Phosphoglycerate kinase (Yeast; 450 U/mg; 10 mg/ml) | 10 | 164 | 18.9   8.5 |
| Malate dehydrogenase (Pig heart; 1450 U/mg; 10 mg/ml) | 3.0 | 158 | 8.2   8.0 |

[a]    These enzymes are all available as 3.2 M ammonium sulfate suspensions from Boehringer-Mannheim Biochemicals (P.O. Box 50414, Indianapolis, IN 46250).

[b]    The stock enzyme mixture contains the indicated volume for each enzyme, centrifuged at 15,000 xg for 10 minutes.

[c]    After centrifugation, the resulting pellets are dissolved in 0.275 ml of the Tris-cysteine buffer as described in the text.

[d]    The final concentration of enzymes contains 250-1000 mCi of carrier-free $^{32}P_i$.

## Table II

| Chemical and Stock Concentration | | Volume used (μl) | Final Concentration in the reaction[b] (mM) |
|---|---|---|---|
| Tris-HCl, pH 9.0 | 1000 mM | 290 | 250 |
| $MgCl_2$ | 1000 mM | 50 | 43 |
| Dithiothreitol | 500 mM | 30 | 13 |
| Dihydroxyacetone ©phosphate (fresh)[a] | 5 mM | 55 | 0.24 |
| ADP (fresh)[a] | 10 mM | 30 | 0.26 |
| Oxaloacetic acid (fresh)[a] | 20 mM | 30 | 0.52 |
| $NAD^+$ (fresh)[a] | 100 mM | 30 | 2.6 |
| $H_2O$ | | 85 | |
| | | 600 μl | |

[a] These chemicals are prepared freshly for each preparation. Oxaloacetic acid is prepared in 50 mM Tris-HCl, pH 7.5. Dithiothreitol solution can be used up to 2 weeks after preparation. All reagents are commercially available: Tris base, $MgCl_2$ and dithiothreitol are obtained from ICN Biomedicals, Inc. (3300 Hyland Avenue, Costa Mesa, CA 92626); dihydroxyacetone as the lithium salt can be obtained from Sigma Chemical Co. (P.O. Box 14508, St. Louis, MO 63178); ADP and oxaloacetic acid can be obtained from Boehringer-Mannheim Biochemicals (P.O. Box 50414, Indianapolis, IN 46250).

[b] The final reaction is in a total volume of 1.16 ml containing 0.6 ml of the reagent mix, 0.06 ml of the enzyme mix (see Table I), and 0.5 ml of $^{32}P_i$ solution.

B. Enzyme Mix. It is preferable to use freshly prepared enzyme mix to initiate the reaction, although the enzyme mix can be used for up to one week after preparation. All of the enzymes used in this reaction can be obtained as an ammonium sulfate suspension from a commercial source (e.g. Boehringer-Mannheim). It is not necessary to remove the salt from the enzyme solutions for the production of [γ-$^{32}$P]ATP. However, it is preferable to remove the sulfate from the reaction if the labeled nucleotide is going to be used directly without purification for preparing other [$^{32}$P]-labeled products (e.g. [α-$^{32}$P]-labeled nucleoside triphosphates). High concentrations of sulfate may affect the reaction of polynucleotide kinase, nuclease, and/or other kinases leading to the formation of [α-$^{32}$P] nucleotides. Sulfate has also been found to inhibit L-α-glycerophosphate dehydrogenase which is used for [γ-$^{32}$P]ATP production with L-α-glycerophosphate as the starting material (Johnson, R.A. and Walseth, T.F., Adv. In Cyclic Nucleotide Res., 10:135-168 (1979)). However, in the unique process of this invention, the dehydrogenase is omitted from the reaction because the starting material is dihydroxyacetone phosphate (see Fig. 1). The solution (34 µl) of enzyme mixture (see Table I) is centrifuged at 15,000 xg for about 10 minutes to remove most of the ammonium sulfate. The resulting pellet is dissolved in about 0.275 ml of 50 mM Tris-HCl (pH 9.0) containing either 20 mM dithiothreitol or cysteine-HCl.

C. Purification of [γ-$^{32}$P]ATP. The labeled nucleotide is first purified by DEAE-Sephadex column chromatography and then by high-performance liquid chromatography. Of several anion-exchange resins tested, DEAE-Sephadex (Pharmacia, A-25, HCOO$^-$ form) has been found to be the most effective for large-scale commercial purification of the nucleotide. After loading the sample onto a DEAE-Sephadex column (1x20 cm), previously washed with about 50 ml of water at 4°C, the column is washed with 100 ml of 0.3 M tetraethyl ammonium bicarbonate (TEAB) to remove P$_i$, AMP and ADP. ATP is then eluted with 0.4 M TEAB. The [γ-$^{32}$P]ATP fractions are pooled and the TEAB is removed by repeated evaporation with methanol. The resulting [γ-$^{32}$P]ATP can be further purified by high-performance liquid chromatography (e.g. surface modified amorphorus silica, Zipax Sax of the DuPont Company). [γ-$^{32}$P]ATP can be eluted from the weak anion-exchange column by TEAB (0.15-0.25 M). After removal of TEAB by repeated evaporation with methanol, [γ-$^{32}$P]ATP can be dissolved in a buffer (e.g. 5-10 mM Tris-HCl, pH 7.5) and stored at -70°C. [γ-$^{32}$P]ATP produced by the above procedure has a specific activity of from about ≧4000 Ci/mmole.

## Example II

Production of [γ-$^{32}$P]dATP from $^{32}$P$_i$. The procedure for preparing [γ-$^{32}$P]dATP is essentially the same as that described above in Example I for [γ-$^{32}$P]ATP, except that 2'-deoxy ADP is substituted for ADP. Reaction and purification of the final product can be carried out with the same techniques described in Example I.

## Example III

Production of [γ-$^{32}$P]GTP from $^{32}$P$_i$. The procedure for preparing [γ-$^{32}$P]GTP is essentially the same as that described above in Example I for [γ-$^{32}$P]ATP, except that GDP is substituted for ADP. Reaction and purification of the final product can be carried out with the same techniques described in Example I.

## Example IV

Production of [γ-$^{32}$P]CTP from $^{32}$P$_i$. The procedure for preparing [γ-$^{32}$P]CTP is essentially the same as that described above in Example I for [γ-$^{32}$P]ATP, except that CDP is substituted for ADP. Reaction and purification of the final product can be carried out with the same techniques described in Example I.

## Example V

Production of [γ-$^{32}$P]dTTP from $^{32}$P$_i$. The procedure for preparing [γ-$^{32}$P]dTTP is essentially the same as that described above in Example I for [γ-$^{32}$P]ATP, except that 2'deoxy TDP is substituted for ADP. Reaction and purification of the final product can be carried out with the same techniques described in Example I.

## Claims

1. A method for preparing [γ-$^{32}$P]-labeled nucleoside triphosphates by enzymatic reactions comprising:
a) combining dihydroxyacetone phosphate (DHAP), [$^{32}$P]-inorganic phosphate ($^{32}$P$_i$), and a nucleoside diphosphate (NDP) into a reaction mixture;
b) adding into the reaction mixture conversion enzymes triosephosphate isomerse (TPI), glyceraldehyde 3-phosphate dehydrogenase (GAPDH) and 3-phosphoglycerate kinase (PGK) to facilitate enzymatic reactions within the reaction mixture; and
c) adding to the reaction mixture during the enzymatic reactions β-nicotinamide adenine dinucleotide (β-NAD$^+$) together with oxaloacetic acid and malate dehydrogenase (MDH) to recycle NAD$^+$ from its reduced form of nicotinamide adenine dinucleotide (NADH).

2. The method of claim 1 in which the nucleoside diphosphate (NDP) is selected from the group consisting of adenosine 5'-diphosphate (ADP), 2'-deoxyadenosine 5'-diphosphate (dADP), guanosine 5'-diphosphate (GDP), cytidine 5'-diphosphate (CDP), and 2'-deoxythymidine 5'-diphosphate (dTDP).

**3.** The method of claim 1 in which the activities of conversion enzymes TPI and GADPH in the reaction mixture, are within the range of from about 2.0 to 3.0 International Activity Units per milliliter (U/ml), to minimize the formation of $^{32}$P-labeled contaminates associated with the conversion enzymes.

**4.** The method of claim 1 in which the ionic strength of the buffer tris-(hydroxymethyl) aminomethane-HCl (Tris-HCl) and the divalent cation magnesium chloride (MgCl$_2$) added to the reaction mixture is maintained from about 200 to 350 mM Tris-HCl (pH 9.0) and from about 30 to 60 mM MgCl$_2$, respectively, to minimize the formation of $^{32}$P-labeled contaminates associated with the reactants and the conversion enzymes.

**4.** The method of claim 1, 3 or 4 in which said NDP is ADP.

**5.** The method of claim 1, 3 or 4 in which said NDP is dADP.

**6.** The method of claim 1, 3 or 4 in which said NDP is GDP.

**7.** The method of claim 1, 3 or 4 in which said NDP is CDP.

**8.** The method of claim 1, 3 or 4 in which said NDP is dTDP.

CH$_2$OH

CH$^2$O

CH$_2$O (P)

DIHYDROXYACETONEPHOSPHATE

TRIOSEPHOSPHATE ISOMERASE (TPI)

CHO

H—C—OH

CH$_2$ (P)

D-GLYCERALDEHYDE 3-PHOSPHATE

$^{32}$PI

NAD$^+$

GLYCERALDEHYDE 3-PHOSPHATE
DEHYDROGENASE (GAPDH)

NADH+H$^+$

OH

$^-$OOC—CH$_2$CH—COO$^-$

MALATE

MALATE
DEHYDROGENASE
(MDH)

COO$^{32}$P

H—C—OH

CH$_2$O (P)

1,3-DIPHOSPHOGLYCERATE

O

$^-$OOC—CH$_2$C—COO$^-$

OXALOACETATE

ADP (or other NDP)
PHOSPHOGLYCERATE KINASE (PGX)

($\gamma$-$^{32}$P)-APT (or other NTP)

COO

H—C—OH

CH$_2$O (P)

3-PHOSPHOGLYCERATE